# FASCICULE DE BREVET EUROPEEN

(11) **EP 4 667 813 B1**
(45) Date de publication et mention de la délivrance du brevet: **02.12.2026**
(21) Numéro de dépôt: 25175797.7
(22) Date de dépôt: 12.05.2025
(51) Int. Cl.: F17C 9/02

(54) **INSTALLATION DE DISTRIBUTION DE GAZ POUR FOURNIR DE L'OXYGÈNE GAZEUX À UN ÉTABLISSEMENT HOSPITALIER**
GASVERTEILUNGSVORRICHTUNG ZUR BEREITSTELLUNG VON GASFÖRMIGEM SAUERSTOFF FÜR KRANKENHAUSANLAGE
GAS DISTRIBUTION PLANT FOR SUPPLYING GASEOUS OXYGEN TO A HOSPITAL FACILITY

(30) Priorité: 20.06.2024 FR 2406613
(43) Date de publication de la demande: 24.12.2025
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR); BOUCHENY, Nicolas, 75007 Paris (FR)
(74) Mandataire: Air Liquide

(56) Documents cités:
- EP-A1- 4 269 861
- US-A1- 2004 146 759
- US-B1- 11 772 969
- US-B2- 11 383 109

## Description

L'invention concerne une installation de distribution de gaz pour fournir de l'oxygène gazeux à un réseau de canalisations d'un établissement hospitalier, tel un hôpital ou analogue.

Dans les établissements hospitaliers, tels les hôpitaux, les cliniques ou autres, il est nécessaire d'amener des gaz médicaux, en particulier de l'air et de l'oxygène (O₂) de qualité médicale, jusqu'aux différents points d'utilisation au sein de ces établissements, par exemple les services de réanimation ou d'urgence, les salles d'opération...

Généralement, l'oxygène est stocké sous forme liquide dans un ou des réservoirs ou capacités de stockage de grande capacité, agencés à l'extérieur de l'établissement hospitalier, qui sont réapprovisionnés par des camions citernes servant à acheminer l'oxygène depuis son site de production jusqu'aux différents établissements hospitaliers à ravitailler. L'oxygène est ensuite distribué aux différents points d'utilisation, c'est-à-dire les différents services hospitaliers, par des conduites, lignes ou canalisations de gaz, de l'établissement hospitalier, couramment appelés « réseau de distribution de gaz » ou « réseau de gaz ». Une installation de ce type est notamment décrite par EP4269861.

Pour diminuer son « empreinte environnementale », un établissement hospitalier doit limiter l'utilisation d'oxygène liquide (LOX) produit par liquéfaction, donc par un procédé de production très énergivore et qui nécessite ensuite une logistique, en particulier des camions citernes pour livrer le LOX, lesquels engendrent de la pollution.

Alternativement, US11772969 propose un système de production d'oxygène alimenté à l'énergie solaire pour les hôpitaux, comprenant une source d'air ; une source de photocatalyseur comprend des points quantiques de phosphore noir ; une chambre de production d'oxygène avec une source de lumière électrique ; une canalisation pour fournir un mélange d'air et de photocatalyseur à la chambre de production d'oxygène ; un panneau solaire photovoltaïque pour fournir de l'énergie électrique à la source de lumière électrique ; un réservoir d'oxygène non filtré pour recevoir et stocker l'oxygène provenant de la chambre de production d'oxygène ; et un réservoir d'hydrogène pour recevoir l'hydrogène provenant de la chambre de production d'oxygène. Un tel système est n'est pas idéal et complexe à mettre en œuvre. Ainsi, la production d'oxygène repose sur la vapeur d'eau contenue dans l'air ambiant, laquelle est en quantité très limitée, ce qui limite la production d'oxygène et/ou requiert un traitement d'un volume d'air très important rendant alors le système encombrant et énergivore. De plus, le photo-catalyseur se dégrade au cours du temps, ce qui nécessite des remplacements réguliers, ce qui impacte les coûts et complexifie le fonctionnement global de l'installation.

Le problème est dès lors de pouvoir disposer d'oxygène pour traiter les patients au sein de l'établissement hospitalier tout en limitant les approvisionnements en LOX qui impactent négativement « l'empreinte environnementale » de l'établissement hospitalier. Autrement dit, le but est de proposer une installation de fourniture d'oxygène gazeux de conception simple, dédiée au milieu hospitalier, et qui permette de produire de l'oxygène sans engendrer ou en limitant les inconvénients susmentionnés.

Une solution selon l'invention porte sur une installation de distribution de gaz pour fournir de l'oxygène gazeux à un réseau de canalisations d'un établissement hospitalier, comprenant une source principale de gaz contenant de l'oxygène, et une ligne principale de gaz venant se raccorder fluidiquement au réseau de canalisations de l'établissement hospitalier, ladite ligne principale de gaz étant raccordée fluidiquement à ladite source principale de gaz.

De plus, l'installation de distribution de gaz comprend par ailleurs :
- au moins un électrolyseur, alimenté en courant électrique et en eau, configuré pour produire au moins de l'oxygène gazeux,
- une ligne d'alimentation additionnelle servant à convoyer de l'oxygène gazeux produit par ledit au moins un électrolyseur,
- un dispositif sélecteur de source de gaz étant agencé sur la ligne principale de gaz et raccordé fluidiquement à ladite ligne d'alimentation additionnelle, et
- une unité de contrôle pilotant ledit dispositif sélecteur de source de gaz de manière à mettre en relation fluidique, le réseau de canalisations de l'établissement hospitalier avec la ligne principale de gaz et/ou avec la ligne d'alimentation additionnelle.

Selon le mode de réalisation considéré, l'installation de distribution de gaz de l'invention peut comprendre l'une ou plusieurs des caractéristiques suivantes :
- selon un mode de réalisation, l'unité de contrôle pilote ledit dispositif sélecteur de source de gaz de manière à mettre en relation fluidique, le réseau de canalisations de l'établissement hospitalier avec simultanément la ligne principale de gaz et la ligne d'alimentation additionnelle pour l'alimenter avec de l'oxygène provenant de la ligne principale de gaz et de la ligne d'alimentation additionnelle. Les proportions/quantités d'oxygène provenant de ces deux lignes peuvent être égales ou différentes d'une ligne à l'autre.
- selon un autre mode de réalisation, l'unité de contrôle pilote ledit dispositif sélecteur de source de gaz de manière à mettre en relation fluidique, le réseau de canalisations de l'établissement hospitalier avec préférentiellement soit la ligne principale de gaz, soit la ligne d'alimentation additionnelle, c'est-à-dire alternativement avec l'une ou l'autre de ces lignes.
- la source principale de gaz comprend un réservoir d'oxygène contenant de l'oxygène sous forme liquide (LOX), alimentant un vaporiseur de gaz permettant de transformer le LOX en oxygène gazeux (GOX).
- le réservoir d'oxygène contient du LOX surmonté d'un ciel gazeux de GOX.
- selon un mode de réalisation, la source principale de gaz contenant de l'oxygène comprend (au moins) une capacité amont contenant du GOX.
- selon un mode de réalisation, la (les) capacité amont contenant du GOX est alimentée en GOX par un générateur d'oxygène produisant de l'oxygène à partir d'air ambiant, de préférence une unité d'adsorption à pression modulée ou unité PSA (pour *Pressure Swing Adsorption)* ou un système à membranes céramiques à transport d'ions ou ITM (pour *Ion Transport Membrane*)*.*
- au moins une capacité de stockage d'oxygène gazeux est agencée en aval dudit au moins un électrolyseur pour stocker tout ou partie de l'oxygène produit par ledit au moins un électrolyseur.
- la ligne d'alimentation additionnelle est raccordée fluidiquement et alimentée en oxygène gazeux par ladite au moins une capacité de stockage d'oxygène gazeux.
- des moyens de mesure de pression sont agencés pour mesurer la pression d'oxygène au sein de ladite au moins une capacité de stockage d'oxygène gazeux.
- les moyens de mesure de pression comprennent un capteur de pression.
- l'unité de contrôle pilote le dispositif sélecteur de source de gaz à partir des mesures de pression opérées par les moyens de mesure de pression.
- l'unité de contrôle comprend au moins une (ou des) carte électronique à microprocesseur(s), i.e. un ou des microprocesseurs.
- le (ou les) microprocesseur(s) met en œuvre un ou des algorithmes.
- un régulateur de pression principal est agencé sur la ligne d'alimentation principale, entre la source principale de gaz et le dispositif sélecteur de source de gaz.
- un régulateur de pression additionnel est agencé entre ledit au moins un électrolyseur et le dispositif sélecteur de source de gaz, en particulier en aval de la capacité de stockage d'oxygène gazeux.
- le régulateur de pression principal et/ou le régulateur de pression additionnel sont configurés pour réduire la pression de l'oxygène gazeux.
- le régulateur de pression principal et/ou le régulateur de pression additionnel sont ou comprennent des dispositifs détendeurs de gaz.
- le régulateur de pression principal et/ou le régulateur de pression additionnel sont configurés pour fournir de l'oxygène gazeux (GOX) à une pression de détente comprise entre 4 et 5 bar (bar relatif), par exemple de l'ordre de 4 bar environ.
- une soupape d'échappement est agencée sur la ligne d'acheminement d'O₂ en aval du (ou des) électrolyseur(s).
- une soupape d'échappement est agencée entre le (ou les) électrolyseur(s) et la capacité de stockage d'oxygène gazeux.
- la soupape d'échappement est configurée pour échapper de l'oxygène gazeux à l'atmosphère lorsque la pression atteint un niveau de pression de sécurité, typiquement au moins 30 bar (bar relatif), i.e. le niveau de pression s'exerçant dans la ligne d'acheminement d'O₂ relié à (ou aux) l'électrolyseur.
- l'unité de télémétrie additionnelle ou secondaire est associée à la capacité de stockage d'O₂ gazeux, par exemple agencée sur la capacité de stockage ou à proximité de celle-ci.
- l'unité de télémétrie additionnelle coopère avec les moyens de mesure de pression mesurant la pression d'oxygène au sein de la capacité de stockage d'oxygène gazeux, i.e. les moyens de mesure de pression fournissent les mesures de pression à l'unité de télémétrie additionnelle.
- l'unité de contrôle comprend un module de télécommunication coopérant avec une unité de télémétrie additionnelle configurée pour transmettre audit module de télécommunication, au moins une partie des mesures de pression d'oxygène opérées par les moyens de mesure de pression.
- ledit au moins un électrolyseur est alimenté en courant électrique par au moins une source d'énergie renouvelable fonctionnant de façon intermittente.
- ladite au moins une source d'énergie renouvelable comprend un ou plusieurs panneaux photovoltaïques, i.e. panneaux solaires.
- elle comprend en outre un dispositif à pile à combustible configuré pour produire du courant électrique à partir d'hydrogène produit par ledit au moins un électrolyseur et fournir au moins une partie du courant électrique produit audit au moins un électrolyseur.
- alternativement, ledit au moins un électrolyseur est alimenté en courant électrique par le réseau, en particulier pendant les périodes de temps où la demande en courant électrique est faible, donc les coûts associés plus bas.
- elle comprend plusieurs électrolyseurs fournissant/produisant chacun de l'oxygène et de l'hydrogène.
- le ou les électrolyseurs sont agencés hors de l'établissement hospitalier, par exemple sur son toit, sur une terrasse, sur une façade, dans une cour ou un espace extérieur, ou autre.
- on utilise au moins une partie du courant électrique (i.e. électricité) produit par la (ou les) source d'énergie renouvelable pour alimenter le (ou les) électrolyseur lorsque la production d'électricité par la (les) source d'énergie renouvelable excède les besoins énergétiques de l'établissement hospitalier, et produire de l'oxygène et de l'hydrogène par électrolyse d'eau au sein dudit (des) électrolyseur(s).
- l'unité de télémétrie secondaire est en outre configurée pour vérifier la conformité de la composition du gaz stocké dans le réservoir secondaire par comparaison à au moins une valeur-seuil mémorisée dans ladite unité de télémétrie secondaire, puis envoyer un signal d'alarme (i.e. alarme de non-conformité) à l'unité de contrôle lorsqu'une non-conformité de composition est détectée.
- ladite au moins une valeur-seuil correspond à une teneur en oxygène et/ou en au moins une impureté choisie parmi H₂O, CO et CO₂. Par exemple, une teneur minimale en oxygène d'au moins 99 vol.%.
- l'unité de contrôle est configurée pour, en réponse à la réception d'un signal d'alarme (i.e. alarme de non-conformité), agir automatiquement sur le sélecteur de source de gaz afin de stopper la fourniture d'oxygène provenant du réservoir secondaire et, par ailleurs, assurer une alimentation en oxygène provenant du conduit d'alimentation principal.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de distribution d'oxygène médical agencée dans un établissement hospitalier selon la présente invention.
Fig. 2 schématise un mode de réalisation d'une unité de gestion de gaz utilisable dans l'installation de distribution de gaz de Fig. 1.

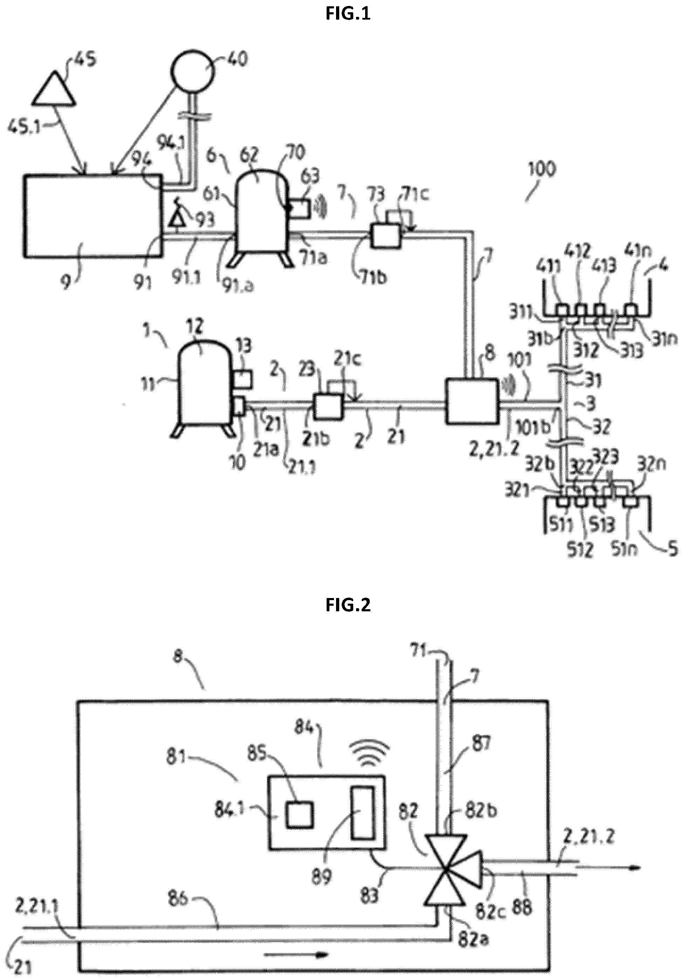

Fig. 1 schématise un mode de réalisation d'une installation de distribution de gaz médical 100, typiquement de fourniture d'oxygène médical, agencée au sein d'un établissement hospitalier, tel un hôpital ou analogue, alimentée par une source principale de gaz 1, à savoir un réservoir principal 11 servant au stockage d'O₂ sous forme liquide dans son volume intérieur 12. Le réservoir principal 11 peut être stocké à l'extérieur de l'établissement hospitalier, par exemple dans une cour arrière ou analogue.

Une unité de télémétrie principale 13 associée à la source principale de gaz 1 mesure en permanence le niveau d'O₂ liquide dans le réservoir principal de stockage 11 et transmet à distance, via un réseau de communication, tel que GSM, internet ou autre, les mesures de niveau d'O₂ liquide à un fournisseur de gaz afin de l'alerter lorsqu'un seuil bas prédéfini, par exemple mémorisé au sein de l'unité 13, est atteint pour qu'il vienne remplir à nouveau le réservoir de stockage principal 11 avec de l'oxygène liquide amené par camion-citerne ou analogue.

Autrement dit, l'unité de télémétrie principale 13 est donc configurée pour être apte à émettre des informations, en particulier des mesures. Pour se faire, elle intègre un module de (télé)communication adapté, notamment un module de communication selon un protocole de type GSM ou analogue.

Afin de transformer l'oxygène liquide en oxygène gazeux, on met en œuvre un vaporiseur de gaz 10 qui est agencé en aval du réservoir principal 11. La sortie du vaporiseur de gaz 10 est reliée fluidiquement (en 21a) au tronçon amont 21.1 d'une ligne d'alimentation principal 2, c'est-à-dire un conduit ou canalisation de gaz principale, de manière à l'alimenter en oxygène gazeux. Le tronçon amont 21.1 alimente quant à lui (en 21b), un régulateur de pression principal 23, typiquement un dispositif détendeur de gaz, agencé sur le conduit d'alimentation principal 2.

Le régulateur de pression principal 23 est par ailleurs relié fluidiquement (en 21c) à un tronçon aval 21.2 de la ligne de gaz principale 2 afin de lui fournir de l'oxygène gazeux à pression donnée. Autrement dit, le régulateur de pression 23 garantit, i.e. fournit, une pression stable dans le tronçon aval 21.2 de la ligne de gaz principale 2, dite « pression nominale de service », laquelle est avantageusement comprise environ entre 4 et 5 bar (dans le cadre de l'invention on utilise simplement « bar » pour désigner une mesure de pression en « bar relatif »), par exemple de l'ordre de 4 bar environ.

L'extrémité aval du tronçon aval 21.2 du conduit principal 2 vient se ramifier (en 101.b) en un premier tronçon ou conduit secondaire 31 et, par ailleurs, en un second tronçon ou conduit secondaire 32. Les premier et second conduits secondaires 31, 32 font partie d'un circuit ou réseau de gaz secondaire 3.

Le premier conduit secondaire 31 présente quant à lui une pluralité de sous-ramifications 311-31n, respectivement, formant des lignes de gaz débouchant au niveau de prises d'oxygène murales 411 à 41n, respectivement, situées dans un service 4 de l'établissement hospitalier, par exemple un service de réanimation ou autre.

De façon similaire, le second conduit secondaire 32 alimente lui aussi une pluralité de sous-ramifications 321-32n, c'est-à-dire des lignes de gaz aboutissant elles aussi à des prises d'O₂ murales 421-42n situées dans un autre service 5 de l'établissement hospitalier, par exemple un service de soins légers ou autre.

L'oxygène gazeux circulant dans le premier conduit secondaire 31 et dans le second conduit secondaire 32 est donc distribué, via les sous-ramifications 311-31n, 321-32n, aux prises d'O₂ murales 411-41n, 421-42n afin d'alimenter un ou des dispositifs médicaux utilisant de l'oxygène, tels que des débitmètres, ventilateurs mécaniques, dispositifs de délivrance d'oxygène à haut débit... ou autres, venant se raccorder fluidiquement auxdites prises d'O₂ murales 411-41n, 421-42n.

Par ailleurs, selon l'invention, l'installation 100 comprend en outre un (ou des) dispositif électrolyseur 9, simplement appelé « électrolyseur », servant à produire de l'oxygène gazeux qui est injecté dans le conduit d'alimentation principal 2, comme expliqué ci-dessous, et par ailleurs de l'hydrogène gazeux. Le (ou chaque) électrolyseur 9 permet de décomposer de l'eau, grâce à de l'énergie électrique qui lui est fournie, pour produire de l'oxygène (O₂) et de l'hydrogène (H₂) gazeux.

L'électrolyseur 9 est préférentiellement raccordé électriquement au secteur électrique, typiquement triphasé, par exemple 3 x 400V (non montré), et/ou au réseau d'eau pour l'alimentation en eau de l'électrolyseur 9.

Il est aussi préférentiellement raccordé 45.1 à une (ou plusieurs) source d'énergie renouvelable 45 qui fonctionne(nt) de façon intermittente. En particulier, l'électrolyseur 9 est configuré pour fonctionner à forte/pleine capacité lorsque la (les) source d'énergie renouvelable 45 produit un excès d'énergie. Préférentiellement, la source d'énergie renouvelable 45 comprend ici un ou plusieurs panneaux photovoltaïques qui peuvent être installés sur le toit ou une façade de l'établissement.

L'électrolyseur 9 est doté d'une sortie d'H₂ 94 pour fournir l'hydrogène produit et, par ailleurs, d'une sortie d'O₂ 91 pour fournir l'oxygène (O₂) produit au sein de l'électrolyseur 9.

La sortie d'H₂ 94 est reliée fluidiquement, via une ligne d'acheminement d'hydrogène 94.1, à un dispositif à pile à combustible 40 servant à générer de l'électricité (lorsque requis) ou, le cas échéant, à un stockage d'H₂ (non montré) servant à stocker l'hydrogène produit et/ou à une ligne d'évacuation de gaz servant à convoyer l'hydrogène produit.

Avantageusement, l'électrolyseur 9 est de type à Membrane Echangeuse de Protons ou PEM (i.e. *Proton Exchange Membrane* en anglais), c'est-à-dire qu'on utilise comme électrolyte, une membrane polymère extrêmement fine, typiquement de 20 à 300 µm d'épaisseur, qui est étanche au gaz et a un caractère fortement acide laissant passer les ions H+ (protons), notamment du fait de groupes fonctionnels sulfonique (R-SO3H) assurant les échange d'ions.

Par contre, en cas de dysfonctionnement ou d'arrêt de la source d'énergie renouvelable 45, le fonctionnement de l'électrolyseur 9 se fait grâce à l'alimentation électrique du secteur et/ou à partir d'électricité produite par le dispositif à pile à combustible 40.

Plus généralement, l'électrolyseur 9 est configuré pour être capable, en fonctionnement, de générer de l'O₂ gazeux à une pression supérieure à 30 bar, typiquement de l'ordre de 33 bar.

De plus, l'électrolyseur 9 dispose préférentiellement de modules internes permettant d'assécher l'O₂ produit pour éliminer les espèces H₂O et, par ailleurs, de le purifier pour éliminer les composés H₂ résiduels éventuellement présents, via un catalyseur adapté par exemple un catalyseur au platine (Pt).

En aval de la sortie d'O₂ 91 de l'électrolyseur 9, i.e. sur la ligne d'acheminement d'O₂ 91.1, est agencée une soupape d'échappement 93 qui est préférentiellement tarée à un niveau de pression de sécurité de 30 bar environ. Elle permet d'envoyer de l'O₂ produit par l'électrolyseur 9 vers l'atmosphère lorsque la pression dans la ligne 91.1 devient supérieure à 30 bar, i.e. qu'elle maintient la pression à environ 30 bar.

La ligne d'acheminement d'O₂ 91.1 alimente (en 91a) une capacité de stockage 6 d'O₂ gazeux, typiquement un réservoir secondaire de gaz 61 sous pression, apte à stocker l'O₂ provenant de l'électrolyseur 9 sous forme gazeuse dans son volume intérieur 62 à une pression de sécurité de 30 bar environ.

Une unité de télémétrie additionnelle ou secondaire 63 associée à la capacité de stockage 6 d'O₂ gazeux, mesure en permanence la pression d'O₂ gazeux dans le réservoir de gaz 61 et transmet à distance, via un réseau de communication, tel que LoRa^{®}, Bluetooth^{®} ou autre, la ou les mesures de pression opérées à une unité de gestion de gaz 8 agencée sur le tronçon aval 21.2 du conduit principal 2, c'est-à-dire entre le régulateur de pression 23 et le site de ramification 101b.

Pour ce faire, on utilise des moyens de mesure de pression 70, tel un capteur de pression (non montré), agencés et configurés pour mesurer la pression d'oxygène gazeux s'exerçant au sein la capacité de stockage 6 d'O₂ gazeux. Les moyens de mesure de pression 70 peuvent être embarqués dans l'unité de télémétrie secondaire 63 ou, le cas échéant, associés à celle-ci pour lui fournir les mesures de pression d'xoygène opérées.

Autrement dit, l'unité de télémétrie secondaire 63 est donc, elle aussi, configurée pour être apte à émettre des informations, en particulier des mesures de pression, et intègre également un module de (télé)communication adapté, notamment un module de communication selon un protocole de type LoRa^{®}, Bluetooth^{®} ou autre ou analogue.

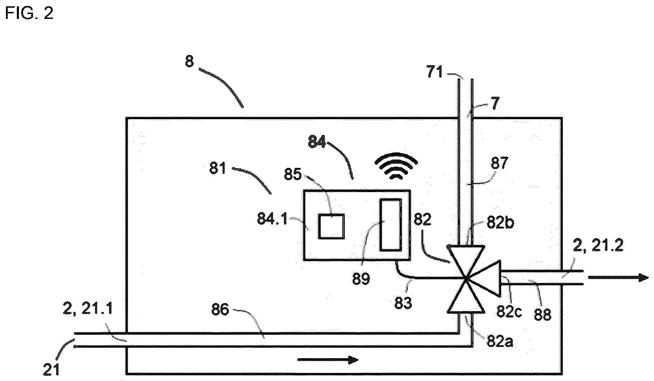

L'unité de télémétrie secondaire 63 comprend par ailleurs, d'un ou de capteurs de concentration(non montrés) mesurant la concentration d'O₂ ainsi que celles d'éventuelles impuretés indésirables, telles que de la vapeur d'eau, du monoxyde de carbone (CO), du dioxyde de carbone (CO₂) et de l'hydrogène (H₂) susceptibles de se trouver dans l'oxygène provenant de la capacité de stockage 6 d'O₂ gazeux afin de vérifier, de préférence en permanence, la pureté de l'O₂ stocké et de s'assurer que sa qualité est conforme notamment aux spécifications réglementaires. En particulier, ceci permet de garantir que la qualité de l'O₂ provenant de la capacité de stockage 6 est égale ou équivalente à celle de l'O₂ fourni par la source d'O₂ liquide stockée dans le réservoir de stockage 11. En cas de déviation de qualité (e.g. concentration non-conforme), l'unité de télémétrie 63 est configurée pour envoyer un signal d'alarme à l'unité de gestion de gaz 8 qui agit en réponse à cette alerte, comme expliqué ci-après en lien avec Fig. 2.

Le réservoir de gaz secondaire 61 est raccordé fluidiquement (en 71a) et alimente une ligne d'alimentation additionnelle 7 servant à convoyer l'oxygène gazeux sortant du réservoir de gaz 61 jusqu'à l'unité de gestion de gaz 8.

La ligne d'alimentation additionnelle 7 comprend par ailleurs un régulateur de pression secondaire ou additionnel 73, typiquement un dispositif détendeur de gaz, agencé entre le réservoir de gaz 61 et l'unité de gestion de gaz 8, en particulier de d'un sélecteur de source de gaz 82. Le détendeur additionnel 73 sert à réguler la pression gazeuse et à fournir une pression stable comprise environ entre 4 et 5 bar (bar relatifs), par exemple de l'ordre de 4 bar environ, c'est-à-dire du même ordre que celle fournie par le régulateur de pression 23 principal fournit également une pression stable dans la portion amont 21, comprise environ entre 4 et 5 bar relatifs, par exemple égale à 4 bar environ.

L'unité de gestion de gaz 8 est donc située sur le tronçon aval 21.2 du conduit principal 2 qui vient se raccorder (i.e. au site de ramification 101b) aux premier et second tronçons 31, 32, comme décrit ci-avant.

Fig. 2 schématise un mode de réalisation de l'unité de gestion de gaz 8 utilisée dans l'installation 100 selon l'invention de Fig. 1.

L'unité de gestion de gaz 8 comprend un sélecteur de source de gaz 82, c'est-à-dire un dispositif de sélection, relié électriquement à une unité de contrôle 84 comprenant une (des) carte électronique 84.1 à microprocesseur(s) 85, tel un (des) microcontrôleur 81, via un câble de connexion 83 ou analogue.

Plus précisément, le sélecteur de source de gaz 82 comprend :
- une première entrée 82a reliée fluidiquement, via un premier conduit d'entrée 86, à une portion amont du tronçon aval 21.2 du conduit d'alimentation principal 21 qui l'alimente en oxygène gazeux sous pression provenant du réservoir de stockage 11.
- une seconde entrée 82b reliée fluidiquement, via un second conduit d'entrée 87, ligne d'alimentation additionnelle 7 qui convoie l'oxygène gazeux sous pression provenant du réservoir de de gaz 61 qui est alimenté par l'électrolyseur 90.
- une sortie 82c reliée fluidiquement, via un conduit de sortie 88, à une portion aval du tronçon aval 21.2 qui vient se raccorder au site de ramification 101b aux premier et second conduits secondaires 31, 32, donc au réseau de distribution de gaz 3 comprenant les conduits secondaires 31, 32.

L'unité de contrôle 84 pilote le sélecteur de source de gaz 82. Plus précisément, le sélecteur de source de gaz 82 est contrôlé par le microprocesseur 85 de l'unité de contrôle 84, via le câble de connexion 83 de manière à autoriser ou interdire une communication fluidique entre l'un ou l'autre des premier et second conduits d'entrée 86, 87 avec le conduit de sortie 88.

Le sélecteur de source de gaz 82 est donc configuré pour diriger l'oxygène gazeux provenant de la source principale de gaz 1, à savoir le réservoir principal 11, ou de la capacité de stockage secondaire 6, i.e. le réservoir secondaire 61, vers les premier et second conduits secondaires 31, 32, via lesdits premier et second conduits d'entrée 86, 87 et conduit de sortie 88.

Autrement dit, le sélecteur de source de gaz 82 opère de manière analogue à une valve 3:2.

L'unité de contrôle 84 comprend en outre un module de télécommunication 89 configuré pour opérer des émissions et/ou des réceptions de données, i.e. informations ou autres. Selon un mode de réalisation, le module de télécommunication 89 est préférentiellement intégré à la carte électronique 84.1 ; toutefois, il peut aussi être dissocié de celle-ci.

Plus précisément, le module de télécommunication 89 est configuré pour communiquer en réception avec l'unité de télémétrie secondaire 63 associée au réservoir secondaire de gaz 61 et/ou en émission avec l'unité de contrôle 84. Ainsi, lorsque le module de télécommunication 89 reçoit des données provenant de l'unité de télémétrie secondaire 63, il les fournit, i.e. les retransmet ou les transfère, à l'unité de contrôle 84 qui les traite.

Tant que le réservoir secondaire 61 n'est pas sous pression suffisante, c'est-à-dire qu'il ne contient pas une quantité d'oxygène minimale donnée assurant le niveau de pression requis, et que l'électrolyseur 90 est en fonctionnement, par exemple du fait d'un surplus d'électricité produit par la (les) source d'énergie renouvelable, le microprocesseur 85 de l'unité de contrôle 84 commande le sélecteur de source de gaz 82 afin de faire communiquer fluidiquement le conduit d'alimentation principal de gaz au réseau de distribution de gaz 3 afin de l'alimenter, via les conduits d'entrée 86 et de sortie 88 du sélecteur 82, avec de l'oxygène provenant uniquement du réservoir de stockage principal 11, c'est-à-dire de la source principale de gaz 1, i.e. d'oxygène.

Pendant que l'électrolyseur 90 est en fonctionnement, il assure une production d'O₂ gazeux qui vient progressivement remplir le réservoir secondaire 61. La pression va donc y augmenter progressivement en étant suivie par l'unité de télémétrie 63 qui opère des mesures de pression en permanence ou périodiquement, et les transmet à l'unité de gestion de gaz 8.

Lorsque la pression dans le réservoir de gaz 61 augmente et atteint une valeur de pression-seuil minimale requise, de préférence d'au moins 10 bar, typiquement d'au moins 12 bar, le microprocesseur 85 de l'unité de contrôle 84 de l'unité de gestion de gaz 8 détecte que la pression-seuil minimale est atteinte ou dépassée, à partir du traitement des mesures transmises par l'unité de télémétrie 63, et peut alors d'agir en réponse à cette détection, sur le sélecteur de source de gaz 82 afin de relier fluidiquement la ligne d'alimentation additionnelle de gaz 7 au réseau de distribution de gaz 3, via les conduits d'entrée 87 et de sortie 88 du sélecteur 82, afin de lui fournir de l'oxygène provenant alors du réservoir secondaire 61 et ce, en lieu et place de celui provenant du réservoir de stockage principal 11.

On comprend que le sélecteur de source de gaz 82 est piloté par l'unité de gestion de gaz 8, en particulier par le microprocesseur 85 de l'unité de contrôle 84, pour autoriser ou interdire la fourniture au réseau 3, d'oxygène gazeux sous pression provenant du réservoir de stockage principal 11 ou, le cas échéant, du réservoir secondaire 61, et ce, à partir des mesures de pression opérées par l'unité de télémétrie 63 associée au réservoir secondaire 61.

Les unités de télémétrie principale 13 et secondaire 63 sont configurées pour être aptes à émettre des informations, en particulier des mesures. Pour se faire, elles intègrent chacune un module de télécommunication.

D'une façon générale, en fonction de la quantité d'O₂ produite par l'électrolyseur 90 et des besoins en O₂, i.e. de la consommation/du soutirage, opérés au sein de l'établissement hospitalier, la pression dans le réservoir secondaire 61 varie au fil du temps. En particulier, elle peut atteindre une pression maximale donnée (i.e. seuil haut), par exemple environ 30 bar, du fait notamment de la soupape d'échappement 93 qui évacue à l'atmosphère tout excès de pression, ou, à l'inverse, progressivement diminuer jusqu'à passer sous un niveau de pression-seuil minimum (i.e. seuil bas) en deçà duquel l'alimentation en oxygène provenant du réservoir secondaire 61 n'est plus possible, i.e. s'interrompt, et celle provenant du réservoir de stockage principal 11 reprend.

Autrement dit, le microprocesseur 85 de l'unité de contrôle 84 est configuré pour (rétro-)agir sur le sélecteur de source de gaz 82 afin de rebasculer l'alimentation en oxygène du réseau 3 sur le conduit d'alimentation principal 2 dès lors que la pression d'oxygène dans le réservoir de gaz secondaire 61 passe sous le seuil de pression minimum donnée, i.e. préfixé et/ou mémorisé, avantageusement le seuil de pression minimum est supérieur ou égale à la pression de détente réglée au niveau du régulateur de pression secondaire 73, à savoir ici 7 bar par exemple ou une pression supérieure à 7 bar. Ce seuil de pression minimum est préférentiellement mémorisé au sein de moyens de mémorisation (non montrés), telle une mémoire flash ou analogue, de l'unité de contrôle 84 ou directement dans le microprocesseur 85 lui-même.

Selon un autre mode de réalisation, le microprocesseur 85 de l'unité de contrôle 84 est configuré pour (rétro-)agir sur le sélecteur de source de gaz 82 afin de contrôler l'alimentation en oxygène du réseau 3 à partir d'oxygène provenant du conduit d'alimentation principal 2, c'est-à-dire du réservoir de stockage principal 11, et de la ligne d'alimentation additionnelle de gaz 7, c'est-à-dire du réservoir secondaire 61. Par exemple, opérer une alimentation proportionnelle en oxygène du réseau 3 provenant en partie du conduit d'alimentation principal 2 et en partie la ligne d'alimentation additionnelle de gaz 7.

Autrement dit, selon l'invention, en contrôlant/pilotant le sélecteur de source de gaz 82, on peut assurer une alimentation du réseau avec de l'oxygène gazeux provenant soit alternativement de l'un ou l'autre des conduit d'alimentation principal 2 et ligne d'alimentation additionnelle de gaz 7, soit simultanément des deux.

D'ailleurs, selon un mode de réalisation particulier, en fonction de la taille de l'électrolyseur 9 et des besoins en oxygène de l'établissement hospitalier considéré, la production d'O₂ par le (ou les) électrolyseur peut être supérieure à la consommation en O₂ de l'établissement et, dès lors, moyennant un réservoir de gaz secondaire 61 dimensionné pour couvrir les besoins journaliers de l'établissement, une fourniture d'O₂ sous forme liquide pourrait ne plus être nécessaire. Dans ce cas, on pourrait même interrompre totalement l'alimentation provenant du conduit d'alimentation principal 2, voire même, à l'extrême, supprimer cette alimentation principale pour alimenter le réseau uniquement avec de l'oxygène produit par le ou les électrolyseurs 9.

Par ailleurs, selon un mode de réalisation, l'unité de télémétrie secondaire 63 peut être aussi configurée pour analyser la composition du gaz stocké dans le réservoir secondaire 61, c'est-à-dire vérifier que sa qualité (i.e. composition) est conforme à celle attendue, par exemple par comparaison à une valeur-seuil (e.g. de concentration en oxygène minimum) mémorisée dans ladite unité de télémétrie secondaire 63 (non montrée). Tant que celle-ci est conforme, elle n'émet pas de signal d'alarme. A titre d'exemple, la valeur-seuil peut être égal à environ 99.5% en volume d'oxygène pour être en conformité avec la pharmacopée européenne, ou à environ 99% en volume d'oxygène pour être en conformité avec la pharmacopée nord-américaine (US).

Bien entendu, l'analyse de la composition du gaz stocké dans le second réservoir 61 peut être étendue à d'autres composés gazeux susceptibles d'être présents dans le gaz stocké et habituellement considérés comme des impuretés, tels que le dioxyde de carbone (CO₂), le monoxyde de carbone (CO) ou bien la vapeur d'eau (H₂O), de manière à s'assurer que les limites en impuretés autorisées (i.e. par les pharmacopées susmentionnées par exemple) sont aussi respectées, i.e. en conformité avec ce qui est attendu. Dans ce cas, une ou des valeurs-seuils additionnelles correspondant à ou aux impuretés considérées, peuvent être aussi mémorisées dans l'unité de télémétrie secondaire 63 par exemple (non montrée).

Lorsqu'une non-conformité de composition est détectée, en particulier une concentration en oxygène insuffisante (i.e. inférieure à la valeur-seuil) et/ou une teneur en impuretés trop forte, l'unité de télémétrie 63 envoie un signal d'alarme à l'unité de contrôle 84.

A la réception de ce signal d'alarme, l'unité de contrôle 84, typiquement son microprocesseur 85, est configurée pour agir automatiquement sur le sélecteur de source de gaz 82 afin de stopper la fourniture d'oxygène provenant du réservoir secondaire 61 et, par ailleurs, assurer une alimentation en oxygène provenant du conduit d'alimentation principal 2, donc du réservoir principal 11, du réseau de distribution de gaz 3 de sorte que les patients ne puissent pas être exposés à de l'oxygène dont la qualité n'est pas conforme aux spécifications fixées, telle la pharmacopée.

D'une façon générale, l'installation 100 de l'invention permet d'utiliser de l'O₂ gazeux produit par un (des) électrolyseur 9 afin de diminuer la consommation de LOX provenant de la source principale d'O₂ liquide 1 et de répondre ainsi au souci d'amélioration de l'empreinte environnementale des établissements hospitaliers.

En effet, utiliser une ou des sources d'énergie renouvelable, tels des panneaux solaires, i.e. photovoltaïques, permet d'assurer tout ou partie du besoin énergétique, i.e. en courant électrique, d'un hôpital.

Toutefois, une source d'énergie renouvelable étant intermittente par nature, la production électrique fluctue dans la journée et peut, à certains moments, excéder les besoins énergétiques de l'établissement. Dès lors, plutôt que de perdre l'excès de courant produit lorsque la production d'électricité par la source d'énergie renouvelable excède les besoins énergétiques de l'établissement, dans le cadre de l'invention, on l'utilise pour alimenter un (ou plusieurs) électrolyseur 9 afin de générer de l'oxygène et de l'hydrogène par électrolyse d'eau, typiquement dans un rapport de 1 :8 (i.e. 1kg d'H₂ pour 8 kg O₂).

Ceci permet non seulement de produire de l'oxygène gazeux (GOX) qui peut être utilisé en lieu et place de LOX, et/ou stocké dans une capacité-tampon optionnelle (non montrée) et par ailleurs d'hydrogène qui peut être stocké, sous forme gazeuse, dans un réservoir à hydrogène dédié, à une pression d'environ 30 bar.

En cas de besoin de courant électrique, par exemple durant la nuit, l'hydrogène stocké peut être transformé en électricité au sein d'une pile à combustible et le courant électrique ainsi produit peut quant à lui servir à alimenter et faire fonctionner l'électrolyseur pour produire notamment de l'oxygène, y compris lorsque les panneaux photovoltaïques ne fonctionnent, par exemple pendant la nuit ou en l'absence de soleil.

Alternativement, notamment durant les périodes de faible demande électrique, typiquement pendant la nuit, on peut aussi alimenter et faire fonctionner l'électrolyseur 9 pour produire notamment de l'oxygène avec de l'électricité provenant du secteur.

## Revendications

1. Installation de distribution de gaz (100) pour fournir de l'oxygène gazeux à un réseau de canalisations (3) d'un établissement hospitalier, comprenant :
- une source principale de gaz (1) contenant de l'oxygène, et
- une ligne principale de gaz (2) venant se raccorder fluidiquement (en 101b) au réseau de canalisations (3) de l'établissement hospitalier, ladite ligne principale de gaz (2) étant raccordée fluidiquement à ladite source principale de gaz (1),
**caractérisée en ce qu'**elle comprend par ailleurs :
- au moins un électrolyseur (9), alimenté en courant électrique et en eau, configuré pour produire au moins de l'oxygène gazeux,
- une ligne d'alimentation additionnelle (7) servant à convoyer de l'oxygène gazeux produit par ledit au moins un électrolyseur (9),
- un dispositif sélecteur de source de gaz (82) étant agencé sur la ligne principale de gaz (2) et raccordé fluidiquement à ladite ligne d'alimentation additionnelle (7), et
- une unité de contrôle (84) pilotant ledit dispositif sélecteur de source de gaz (82) de manière à mettre en relation fluidique, le réseau de canalisations (3) de l'établissement hospitalier avec la ligne principale de gaz (2) et/ou la ligne d'alimentation additionnelle (7).

2. Installation selon la revendication 1, **caractérisée en ce que** la source principale de gaz (1) comprend un réservoir d'oxygène (11) contenant de l'oxygène sous forme liquide (LOX), alimentant un vaporiseur de gaz (10) permettant de transformer le LOX en oxygène gazeux (GOX).

3. Installation selon la revendication 1, **caractérisée en ce qu'**au moins une capacité de stockage d'oxygène gazeux (6) est agencée en aval dudit au moins un électrolyseur (9) pour stocker tout ou partie de l'oxygène produit par ledit au moins un électrolyseur (9).

4. Installation selon les revendications 1 et 3, **caractérisée en ce que** la ligne d'alimentation additionnelle (7) est raccordée fluidiquement et alimentée en oxygène gazeux par ladite au moins une capacité de stockage d'oxygène gazeux (6).

5. Installation selon les revendications 1 et 3, **caractérisée en ce que** :
- des moyens de mesure de pression (70) sont agencés pour mesurer la pression d'oxygène au sein de ladite au moins une capacité de stockage d'oxygène gazeux (6), et
- l'unité de contrôle (84) pilote le dispositif sélecteur de source de gaz (82) à partir des mesures de pression opérées par les moyens de mesure de pression (70).

6. Installation selon les revendications 1 et 3, **caractérisée en ce que**
- un régulateur de pression principal (23) est agencé sur la ligne d'alimentation principale (2), entre la source principale de gaz (1) et le dispositif sélecteur de source de gaz (82), et/ou
- un régulateur de pression additionnel (73) est agencé entre ledit au moins un électrolyseur (9) et le dispositif sélecteur de source de gaz (82), en particulier en aval de la capacité de stockage d'oxygène gazeux (6).

7. Installation selon les revendications 1 et 5, **caractérisée en ce que** l'unité de contrôle (84) comprend un module de télécommunication (89) coopérant avec une unité de télémétrie (63) additionnelle configurée pour transmettre audit module de télécommunication (89), au moins une partie des mesures de pression d'oxygène opérées par les moyens de mesure de pression (70).

8. Installation selon la revendication 1, **caractérisée en ce que** ledit au moins un électrolyseur (9) est alimenté en courant électrique par au moins une source d'énergie renouvelable (45) fonctionnant de façon intermittente.

9. Installation selon la revendication 8, **caractérisée en ce que** ladite au moins une source d'énergie renouvelable (45) comprend un ou plusieurs panneaux photovoltaïques.

10. Installation selon la revendication 1, **caractérisée en ce qu'**elle comprend en outre un dispositif à pile à combustible (40) configuré pour produire du courant électrique à partir d'hydrogène produit par ledit au moins un électrolyseur (9) et fournir au moins une partie du courant électrique produit audit au moins un électrolyseur (9).

11. Installation selon la revendication 1, **caractérisée en ce que** ledit au moins un électrolyseur (9) est de type à membrane échangeuse de protons (PEM).

12. Installation selon la revendication 1, **caractérisée en ce qu'**une soupape d'échappement (93) est agencée sur la ligne d'acheminement d'O₂ en aval dudit au moins un électrolyseur (9).

13. Installation selon les revendications 3 et 12, **caractérisée en ce que** la soupape d'échappement (93) est agencée entre ledit au moins un électrolyseur (9) et la capacité de stockage d'oxygène gazeux (6).

14. Installation selon l'une des revendications 1 ou 5, **caractérisée en ce que** l'unité de contrôle (84) comprend au moins une carte électronique (84.1) à microprocesseur (85).

15. Installation selon la revendication 1, **caractérisée en ce que** :
- l'unité de télémétrie secondaire (63) est en outre configurée pour :
a) vérifier la conformité de la composition du gaz stocké dans le réservoir secondaire (61) par comparaison à au moins une valeur-seuil mémorisée dans ladite unité de télémétrie secondaire (63), et
b) envoyer un signal d'alarme à l'unité de contrôle (84) lorsqu'une non-conformité de composition est détectée, et
- l'unité de contrôle (84) est configurée pour, en réponse à la réception dudit signal d'alarme, agir automatiquement sur le sélecteur de source de gaz (82) afin de stopper la fourniture d'oxygène provenant du réservoir secondaire (61) et, par ailleurs, assurer une alimentation en oxygène provenant du conduit d'alimentation principal (2).

## Patentansprüche

1. Gasverteilungsanlage (100) zur Zufuhr von gasförmigem Sauerstoff zu einem Rohrleitungsnetz (3) einer Krankenhauseinrichtung, umfassend:
- eine Hauptgasquelle (1), die Sauerstoff enthält, und
- eine Hauptgasleitung (2), die zur fluidischen Verbindung (bei 101b) mit dem Rohrleitungsnetz (3) der Krankenhauseinrichtung kommt, wobei die besagte Hauptgasleitung (2) fluidisch mit der besagten Hauptgasquelle (1) verbunden ist,
**dadurch gekennzeichnet, dass** sie ferner umfasst:
- mindestens einen Elektrolyseur (9), der mit elektrischem Strom und Wasser versorgt wird und so konfiguriert ist, dass er mindestens gasförmigen Sauerstoff erzeugt,
- eine zusätzliche Versorgungsleitung (7), die dazu dient, den von dem besagten mindestens einen Elektrolyseur (9) erzeugten gasförmigen Sauerstoff zu fördern,
- eine Gasquellenauswahlvorrichtung (82), die an der Hauptgasleitung (2) angeordnet und fluidisch mit der besagten zusätzlichen Versorgungsleitung (7) verbunden ist, und
- eine Steuereinheit (84), die die besagte Gasquellenauswahlvorrichtung (82) steuert, um das Rohrleitungsnetz (3) der Krankenhauseinrichtung mit der Hauptgasleitung (2) und/oder der zusätzlichen Versorgungsleitung (7) in fluidische Verbindung zu bringen.

2. Anlage nach Anspruch 1, **dadurch gekennzeichnet, dass** die Hauptgasquelle (1) einen Sauerstoffbehälter (11) umfasst, der Sauerstoff in flüssiger Form (LOX) enthält und einen Gasverdampfer (10) speist, der es ermöglicht, den LOX in gasförmigen Sauerstoff (GOX) umzuwandeln.

3. Anlage nach Anspruch 1, **dadurch gekennzeichnet, dass** mindestens eine Speicherkapazität für gasförmigen Sauerstoff (6) stromabwärts des besagten mindestens einen Elektrolyseurs (9) angeordnet ist, um den gesamten oder einen Teil des von dem besagten mindestens einen Elektrolyseur (9) erzeugten Sauerstoffs zu speichern.

4. Anlage nach den Ansprüchen 1 und 3, **dadurch gekennzeichnet, dass** die zusätzliche Versorgungsleitung (7) fluidisch mit der besagten mindestens einen Speicherkapazität für gasförmigen Sauerstoff (6) verbunden ist und von dieser mit gasförmigem Sauerstoff versorgt wird.

5. Anlage nach den Ansprüchen 1 und 3, **dadurch gekennzeichnet, dass**:
- Druckmessmittel (70) angeordnet sind, um den Sauerstoffdruck innerhalb der besagten mindestens einen Speicherkapazität für gasförmigen Sauerstoff (6) zu messen, und
- die Steuereinheit (84) die Gasquellenauswahlvorrichtung (82) auf der Grundlage der von den Druckmessmitteln (70) durchgeführten Druckmessungen steuert.

6. Anlage nach den Ansprüchen 1 und 3, **dadurch gekennzeichnet, dass**
- ein Hauptdruckregler (23) an der Hauptversorgungsleitung (2) zwischen der Hauptgasquelle (1) und der Gasquellenauswahlvorrichtung (82) angeordnet ist, und/oder
- ein zusätzlicher Druckregler (73) zwischen dem besagten mindestens einen Elektrolyseur (9) und der Gasquellenauswahlvorrichtung (82) angeordnet ist, insbesondere stromabwärts der Speicherkapazität für gasförmigen Sauerstoff (6).

7. Anlage nach den Ansprüchen 1 und 5, **dadurch gekennzeichnet, dass** die Steuereinheit (84) ein Telekommunikationsmodul (89) umfasst, das mit einer zusätzlichen Telemetrieeinheit (63) zusammenwirkt, die konfiguriert ist, um an das besagte Telekommunikationsmodul (89) mindestens einen Teil der von den Druckmessmitteln (70) durchgeführten Sauerstoffdruckmessungen zu übertragen.

8. Anlage nach Anspruch 1, **dadurch gekennzeichnet, dass** der besagte mindestens eine Elektrolyseur (9) mit elektrischem Strom von mindestens einer erneuerbaren Energiequelle (45) versorgt wird, die intermittierend arbeitet.

9. Anlage nach Anspruch 8, **dadurch gekennzeichnet, dass** die besagte mindestens eine erneuerbare Energiequelle (45) ein oder mehrere Photovoltaikpaneele umfasst.

10. Anlage nach Anspruch 1, **dadurch gekennzeichnet, dass** sie ferner eine Brennstoffzellenvorrichtung (40) umfasst, die konfiguriert ist, um elektrischen Strom aus Wasserstoff zu erzeugen, der von dem besagten mindestens einen Elektrolyseur (9) erzeugt wird, und um mindestens einen Teil des erzeugten elektrischen Stroms dem besagten mindestens einen Elektrolyseur (9) zuzuführen.

11. Anlage nach Anspruch 1, **dadurch gekennzeichnet, dass** der besagte mindestens eine Elektrolyseur (9) vom Typ einer Protonenaustauschmembran (PEM) ist.

12. Anlage nach Anspruch 1, **dadurch gekennzeichnet, dass** ein Auslassventil (93) an der O2-Förderleitung stromabwärts des besagten mindestens einen Elektrolyseurs (9) angeordnet ist.

13. Anlage nach den Ansprüchen 3 und 12, **dadurch gekennzeichnet, dass** das Auslassventil (93) zwischen dem besagten mindestens einen Elektrolyseur (9) und der Speicherkapazität für gasförmigen Sauerstoff (6) angeordnet ist.

14. Anlage nach einem der Ansprüche 1 oder 5, **dadurch gekennzeichnet, dass** die Steuereinheit (84) mindestens eine Elektronikplatine (84.1) mit einem Mikroprozessor (85) umfasst.

15. Anlage nach Anspruch 1, **dadurch gekennzeichnet, dass**:
- die sekundäre Telemetrieeinheit (63) ferner konfiguriert ist, um:
a) die Konformität der Zusammensetzung des im sekundären Behälter (61) gespeicherten Gases durch Vergleich mit mindestens einem in der besagten sekundären Telemetrieeinheit (63) gespeicherten Schwellenwert zu überprüfen, und
b) ein Alarmsignal an die Steuereinheit (84) zu senden, wenn eine Nichtkonformität der Zusammensetzung festgestellt wird, und
- die Steuereinheit (84) so konfiguriert ist, dass sie als Reaktion auf den Empfang des besagten Alarmsignals automatisch auf den Gasquellenauswähler (82) einwirkt, um die Zufuhr von Sauerstoff aus dem sekundären Behälter (61) zu stoppen und darüber hinaus eine Versorgung mit Sauerstoff aus der Hauptversorgungsleitung (2) sicherzustellen.

## Claims

1. A gas distribution installation (100) for supplying gaseous oxygen to a pipe network (3) of a hospital establishment, comprising:
- a main gas source (1) containing oxygen, and
- a main gas line (2) for fluidly connecting (at 101b) to the pipe network (3) of the hospital establishment, said main gas line (2) being fluidly connected to said main gas source (1),
**characterized in that** it further comprises:
- at least one electrolyzer (9), supplied with electric current and water, configured to produce at least gaseous oxygen,
- an additional supply line (7) for conveying gaseous oxygen produced by said at least one electrolyzer (9),
- a gas source selector device (82) being arranged on the main gas line (2) and fluidly connected to said additional supply line (7), and
- a control unit (84) controlling said gas source selector device (82) so as to place in fluid communication the pipe network (3) of the hospital establishment with the main gas line (2) and/or the additional supply line (7).

2. The installation according to claim 1, **characterized in that** the main gas source (1) comprises an oxygen tank (11) containing oxygen in liquid form (LOX), supplying a gas vaporizer (10) for transforming the LOX into gaseous oxygen (GOX).

3. The installation according to claim 1, **characterized in that** at least one gaseous oxygen storage capacity (6) is arranged downstream of said at least one electrolyzer (9) to store all or part of the oxygen produced by said at least one electrolyzer (9).

4. The installation according to claims 1 and 3, **characterized in that** the additional supply line (7) is fluidly connected to and supplied with gaseous oxygen by said at least one gaseous oxygen storage capacity (6).

5. The installation according to claims 1 and 3, **characterized in that**:
- pressure measurement means (70) are arranged to measure the oxygen pressure within said at least one gaseous oxygen storage capacity (6), and
- the control unit (84) controls the gas source selector device (82) based on the pressure measurements made by the pressure measurement means (70).

6. The installation according to claims 1 and 3, **characterized in that**
- a main pressure regulator (23) is arranged on the main supply line (2), between the main gas source (1) and the gas source selector device (82), and/or
- an additional pressure regulator (73) is arranged between said at least one electrolyzer (9) and the gas source selector device (82), in particular downstream of the gaseous oxygen storage capacity (6).

7. The installation according to claims 1 and 5, **characterized in that** the control unit (84) comprises a telecommunication module (89) cooperating with an additional telemetry unit (63) configured to transmit to said telecommunication module (89), at least a portion of the oxygen pressure measurements made by the pressure measurement means (70).

8. The installation according to claim 1, **characterized in that** said at least one electrolyzer (9) is supplied with electric current by at least one renewable energy source (45) operating intermittently.

9. The installation according to claim 8, **characterized in that** said at least one renewable energy source (45) comprises one or more photovoltaic panels.

10. The installation according to claim 1, **characterized in that** it further comprises a fuel cell device (40) configured to produce electric current from hydrogen produced by said at least one electrolyzer (9) and to supply at least a part of the electric current produced to said at least one electrolyzer (9).

11. The installation according to claim 1, **characterized in that** said at least one electrolyzer (9) is of the proton exchange membrane (PEM) type.

12. The installation according to claim 1, **characterized in that** an exhaust valve (93) is arranged on the O2 delivery line downstream of said at least one electrolyzer (9).

13. The installation according to claims 3 and 12, **characterized in that** the exhaust valve (93) is arranged between said at least one electrolyzer (9) and the gaseous oxygen storage capacity (6).

14. The installation according to any one of claims 1 or 5, **characterized in that** the control unit (84) comprises at least one electronic board (84.1) with a microprocessor (85).

15. The installation according to claim 1, **characterized in that**:
- the secondary telemetry unit (63) is further configured to:
a) verify the conformity of the composition of the gas stored in the secondary reservoir (61) by comparison with at least one threshold value stored in said secondary telemetry unit (63), and
b) send an alarm signal to the control unit (84) when a non-conformity of composition is detected, and
- the control unit (84) is configured to, in response to receiving said alarm signal, act automatically on the gas source selector (82) to stop the supply of oxygen from the secondary reservoir (61) and, moreover, to ensure a supply of oxygen from the main supply line (2).
